(19) Europäisches Patentamt
European Patent Office
Office européen des brevets

(11) Publication number : 0 512 813 A2

(12) **EUROPEAN PATENT APPLICATION**

(21) Application number : 92304075.2

(51) Int. Cl.$^5$ : C07D 463/00

(22) Date of filing : 06.05.92

(30) Priority : 10.05.91 US 698427

(43) Date of publication of application : 11.11.92 Bulletin 92/46

(84) Designated Contracting States : AT BE CH DE DK ES FR GB GR IT LI LU NL PT SE

(71) Applicant : ELI LILLY AND COMPANY
Lilly Corporate Center
Indianapolis Indiana 46285 (US)

(72) Inventor : Berglund, Richard Alan
4208 Oak Hill Drive
Lafayette, Indiana 47905 (US)

(74) Representative : Hudson, Christopher Mark et al
Erl Wood Manor
Windlesham Surrey GU20 6PH (GB)

(54) **Process for preparing doubly blocked 1-carba(1-dethia)-3-cephem-4-carboxylic acids.**

(57) A process is provided for producing 7-protected-amino-4-protected carboxy-3-halo-1-carbacephalosporins which includes the steps of mixing the hydrochloride salt of the carbacephalosporin with a polar organic aprotic solvent having a dielectric constant of at least about 20 and a base selected from a group consisting of 5- or 6-membered tertiary cyclic amine containing 0-1 oxygen atoms, and dimethylbenzamine to form the free amine of a carbacephalosporin and thereafter without isolating the free amine mixing it with an acylating agent to result in the doubly blocked carbacephalosporin.

EP 0 512 813 A2

This invention relates to a process for preparing intermediates for β-lactam antibiotics, and more particularly to a process for preparing doubly blocked 7-amino -1-carba(1-dethia)-3-cepham-4-carboxylic acids.

The 1-carba(1-dethia)-3-cephem-4-carboxylic acids, hereinafter 1-carbacephalosporins, or carbacephs, possess the 4,6-bicyclic ring system represented by the following structural formula

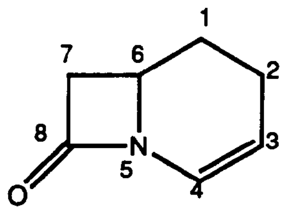

wherein the numbering system is that commonly employed according to the arbitrary cephem nomenclature system.

Hashimoto et al., in U.S. Patent No. 4,335,211, incorporated herein by reference, disclose a class of 1-carbacephalosporins having desirable antibiotic and oral activity characteristics. These compounds are currently being evaluated for the treatment of various conditions such as common upper and lower respiratory tract infections caused by the pathogen H. influenza. one such compound, 7-(R)-phenylglycinamido-3-chloro-1-azidbicyclo(4,2,0)oct-2-ene-8-one-2-carboxylic acid, known as Loracarbef, below,

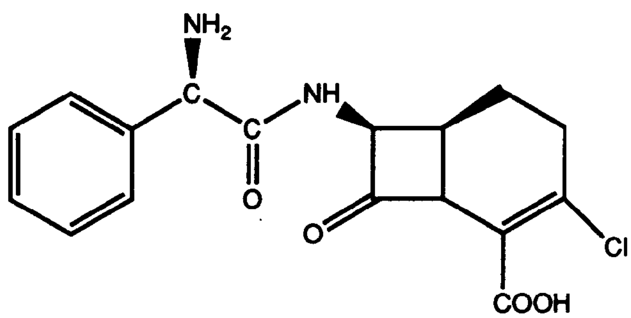

has shown activity against a broad spectrum of bacteria in laboratory tests. Loracarbef has proven to be a relatively stable compound which exhibits high blood levels and relatively long half life.

The 1-carbacephalosporins thus far have not been obtained from natural sources, for example, as microbial metabolites. Despite considerable synthetic difficulties in forming these compounds, in view of their pharmacological properties there is presently intense interest in such carbacephalosporins. Accordingly, methods for the total synthesis of these promising compounds and intermediates to these compounds are highly desirable, particularly methods which are adaptable to large scale manufacture, and result in high yields and reduced cost of manufacture.

One of the more noteworthy approaches to total synthesis of 1-carbacephalosporins is the asymmetric route described by Evans et al., U.S. Patent No. 4,665,171. The preparation of 1-carbacephalosporanic acids and C-3 substituted methyl derivatives thereof is taught broadly by Christenson et al., U.S. Patent No. 4,226,866. Hirata et al., U.K. Patent Application No. 2041923 teach a process for preparing 3-halo and 3-H 1-carbacephalosporins; and Hatanaka et al., Tetrahedron Letters, Vol. 24, No. 44, pages 4037-4038 (1983), teach a process for preparing 3-hydroxy-(+/-)-1-carbacephalosporins.

In accordance with the invention, a process is provided for the preparation of compounds having the formula (1)

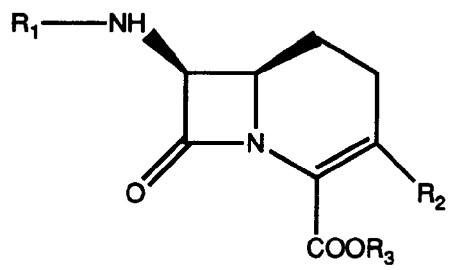

(1)

wherein $R_2$ is hydrogen, trihalo ($C_1$-$C_4$ alkyl) , $C_1$-$C_4$ alkyl, $C_1$-$C_4$ substituted alkyl, $C_1$-$C_4$ alkoxy, $C_1$-$C_4$ substituted alkoxy, $C_1$-$C_6$ alkylthio, $C_1$-$C_6$ substituted alkylthio, methoxy methyl, carbamoyloxy methyl, acetoxymethyl, $C_2$-$C_6$ alkenyl, $C_2$-$C_6$ substituted alkenyl, or halogen;

$R_3$ is a carboxy-protecting group; and

$R_1$ is a group of the formula

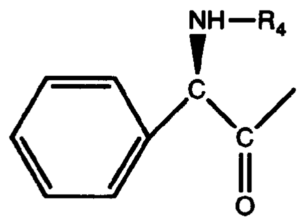

wherein $R_4$ is hydrogen or an amino-protecting group; which includes, under substantially anhydrous conditions, the steps of mixing the compound of the formula

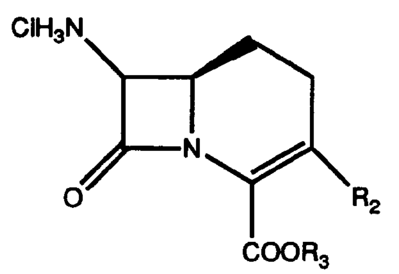

(2)

with a polar organic aprotic solvent having a dielectric constant of at least about 20 and a 5- or 6-membered tertiary cyclic amine base containing 0-1 oxygen atoms or dimethylbenzylamine, at a temperature and for a time sufficient to form the free amine of the compound of formula (2), and thereafter, without isolating the free amine, mixing the free amine with an acylating agent of the formula

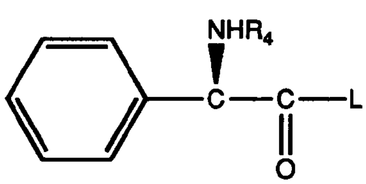

wherein L is a leaving group, for a time and at a temperature sufficient to form compounds of the formula (1).

The invention, together with its objects and advantages thereof, may be best understood by reference to the following description. However, it should be understood that the invention may be embodied in other specific forms without departing from the spirit or central characteristics thereof.

The term "halogen" includes bromo, chloro, fluoro, and iodo.

The term "carboxy-protecting group" as used in the specification refers to one of the ester derivatives of a carboxylic acid group commonly employed to block or protect the carboxylic acid group while reactions are carried out on other functional groups on the compound. Examples of such carboxylic acid-protecting groups include allyl, 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4′-dimethoxybenzhydryl, 2,2′4,4′-tetramethoxybenzhydryl, t-butyl, t-amyl, trityl, 4-methoxytrityl, 4,4′-dimethoxytrityl, 4,4′,4″-trimethoxytrityl, 2-phenyl-prop-2-yl, trimethylsilyl, t-butyldimethylsilyl, phenacyl, 2,2,2-trichloroethyl, b-(tri-methylsilyl)ethyl, b-(di(n-butyl)methylsilyl)ethyl, p-toluenesulfonylethyl, 4-nitrobenzylsulfonylethyl, allyl, cinnamyl, 1-(trimethylsilylmethyl)prop-1-en-3-yl, and like moieties. The species of carboxy-protecting group employed is not critical so long as the derivatized carboxylic acid is stable to the condition of subsequent reaction(s) on other positions of the molecule and can be removed at the appropriate point without disrupting the remainder of the molecule. In particular, it is important not to subject the carboxy-protecting molecule to strong nucleophilic bases. Further examples of these groups are found in E. Haslam, "Protective Groups in organic Chemistry", J. G. W. McOmie, Ed., Plenum Press, New York, NY, 1973, Chapter 5, and T. W. Greene, "Protective Groups in Organic Synthesis", John Wiley and Sons, New York, NY, 1981, Chapter 5. The related term "protected carboxy" denotes that a carboxy group is substituted with one of the above carboxy-protecting groups.

The term "amino-protecting group" refers to substituents of the amino group commonly employed to block or protect the amino functionality while reacting other functional groups on the compound. Examples of such amino-protecting groups include acylvinyl amines such as those enamines derived from ($C_1$-$C_4$ alkyl) acetoacetates, the formyl group, the trityl group, the phthalimido group, the trichloroacetyl group, the chloroacetyl, bromoacetyl, iodoacetyl, phenoxyacetyl and phenylacetyl groups, urethane-type blocking groups such as benzyloxycarbonyl, allyloxycarbonyl 4-phenylbenzyl-oxycarbonyl, 2-methylbenzyloxycarbonyl, 4-methoxybenzyloxycarbonyl, 4-fluorobenzyloxycarbonyl, 4-chlorobenzyloxycarbonyl, 3-chlorobenzyloxycarbonyl, 2-chlorobenzyloxycarbonyl, 2,4-dichlorobenzyloxycarbonyl, 4-bromobenzyloxycarbonyl, 3-bromobenzyloxycarbonyl, 4-nitrobenzyloxycarbonyl, 4-cyanobenzyloxycarbonyl, 2-(4-xenyl)isopropoxycarbonyl, 1,1-diphenyleth-1-yloxycarbonyl, 1,1-diphenyl-prop-1-yloxycarbonyl, 2-phenylprop-2-xyloxycarbonyl, 2-(p-toluyl)prop-2-yloxycarbonyl, cyclopentyloxycarbonyl, 1-methylcyclopentyloxycarbonyl, cyclohexyloxycarbonyl, 1-methylcyclohexyloxycarbonyl, 2-methylcyclohexyloxycarbonyl, 2-(4-toluylsulfonyl)ethoxycarbonyl, 2-(methylsulfonyl)-ethoxycarbonyl, 2-(triphenylphosphino)-ethoxycarbonyl, 9-fluorenylmethoxycarbonyl ("FMOC"), 2-(trimethylsilyl)-ethoxycarbonyl, allyloxycarbonyl, 1-(trimethylsilylmethyl)-prop-1-enyloxycarbonyl, 5-benzisoxalylmethoxycarbonyl, 4-acetoxybenzyloxycarbonyl, 2,2,2-trichloroethoxycarbonyl, 2-ethynyl-2-propoxycarbonyl, cyclopropylmethoxycarbonyl, 4-(decycloxy)benzyloxycarbonyl, isobornyloxycarbonyl, 1-piperidyloxycarbonyl, and the like; the benzoylmethylsulfonyl group, the 2-(nitro)phenylsulfenyl group, the diphenylphosphine oxide group, and like amino-protecting groups. The species of amino-protecting group employed is not critical so long as the derivatized amino group is stable to the condition of subsequent reaction(s) on other positions of the molecule and can be removed at the appropriate point without disrupting the remainder of the molecule. Preferred amino-protecting groups are the t-butoxycarbonyl, phenoxyacetyl, and enamines derived from ($C_1$-$C_4$ alkyl)acetoacetates groups. Similar amino-protecting groups used in the cephalosporin, penicillin and peptide art are also embraced by the above terms. Further examples of groups referred to by the above terms are described by J. W. Barton, "Protective Groups in organic Chemistry", J. G. W. McOmie, Ed., Plenum Press, New York, NY, 1973, Chapter 2, and T. W. Greene, "Protective Groups in organic Synthesis", John Wiley and Sons, New York, NY, 1981, Chapter 7. The related term "protected aminoil denotes that an amino is substituted with an amino-protecting group discussed above.

The term $C_1$-$C_4$ alkyl refers to the straight and branched chain alkyl groups such as methyl, ethyl, n-propyl, isopropyl, n-butyl, t-butyl, and like alkyl groups. The term $C_1$-$C_4$ substituted alkyl refers to $C_1$-$C_4$ alkyl having substituents such as cyano, carboxy, halogen, amino, alkoxy, alkylthio, trifluoromethyl, and trifluoromethylthio. For example, $C_1$-$C_4$ alkyl substituted by cyano refers to cyanomethyl, cyanoethyl, 4-cyanobutyl, and the like; $C_1$-$C_4$ alkyl substituted by carboxy refers to such groups as carboxymethyl, 2-carboxyethyl, 2-carboxypropyl, 4-carboxybutyl, and the like; $C_1$-$C_4$ alkyl substituted by halogen refers to chloromethyl, bromomethyl, 2-chloroethyl, 1-bromoethyl, 4-chlorobutyl, 4-fluorobutyl, 3-fluoropropyl, fluoromethyl, and the like; $C_1$-$C_4$ alkyl substituted by amino refers to such groups as 2-aminoethyl, aminomethyl, 3-aminopropyl and 4-aminobutyl; $C_1$-$C_4$ alkyl substituted by $C_1$-$C_4$ alkoxy refers to methoxymethyl, 2-methoxyethyl, 2-ethoxyethyl,ethoxymethyl, 3-propoxypropyl, 3-ethoxybutyl, 4-t-butyloxybutyl, and like groups; $C_1$-$C_4$ alkyl substituted by $C_1$-$C_4$-alkylthio refers to such groups as for example methylthiomethyl, 2-methylthioethyl, 2-ethylthiopropyl, 4-methylthiobutyl, 3-t-butylthiopropyl, and like groups; $C_1$-$C_4$ alkyl substituted by trifluoromethyl is exemplified by 2,2,2-trifluoroethyl, 3,3,3-trifluoropropyl, 4,4,4-trifluorobutyl, and the like; and $C_1$-$C_4$ alkyl substituted by trifluoromethylthio refers to, for example, trifluoromethylthiomethyl, 2-trifluoromethylthioethyl, 2-trifluoromethylthiopropyl, 4-trifluoromethylthiobutyl, and like $C_1$-$C_4$ alkyl substituted groups.

The term $C_1$-$C_6$ alkylthio and $C_1$-$C_6$ substituted alkylthio denote $C_1$-$C_6$ alkyl and substituted alkyl groups which are attached to a sulfur.

The term $C_1$-$C_4$ alkoxy denotes groups such as methoxy, n-propoxy, ethoxy and the like. The term $C_1$-$C_4$

substituted alkoxy denotes $C_1$-$C_4$ alkoxy groups substituted with such groups as cyano, carboxy, halogen, amino, and alkylthio.

The term $C_2$-$C_6$ alkenyl denotes such groups as vinyl, 1-propenyl, methyl ethenyl, and the like. The term $C_1$-$C_4$ substituted alkenyl denotes $C_1$-$C_4$ alkenyls substituted with such groups as halogen, $C_1$-$C_4$ alkoxy, hydroxy, nitro, or trihalo ($C_1$-$C_4$ alkyl) .

The substituent L is a group which, under the reaction conditions will leave, allowing the free amine to bond to the carbonyl group. Leaving groups include those where L is of the formula

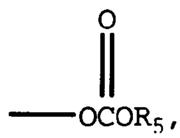

where $R_5$ is $C_1$-$C_6$ alkyl, or L is Cl; Br; I; active esters such as p-nitrophenyl; the adducts of dicyclohexylcarbodiimide, of the general formula

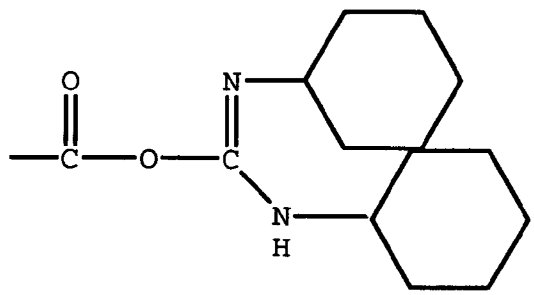

;

And $N_3$. A preferred leaving group is

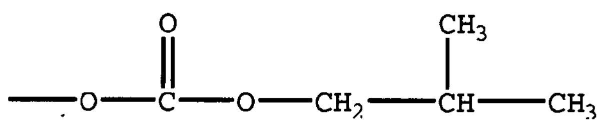

.

The following scheme illustrates one embodiment of the invention (PNB = para-nitrobenzyl).

**SCHEME**

(I)

(IIA)

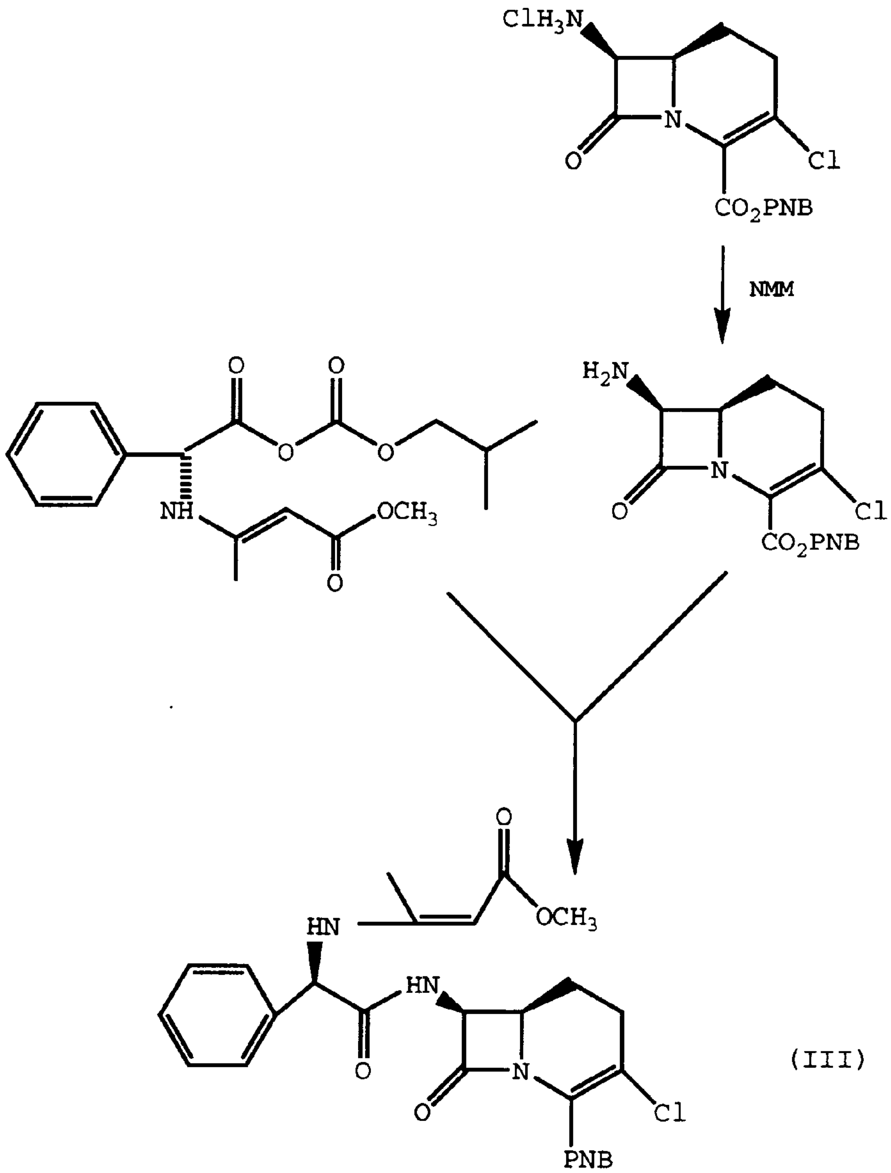


(IIB)

(III)

As noted previously, the process is carried out under substantially anhydrous conditions. Trace amounts of water are tolerable; however, it is desirable to maintain the reaction in the process as dry as possible.

The polar organic aprotic solvent should have a dielectric constant of at least about twenty. Such polar organic aprotic solvents having a dielectric constant of at least about twenty include dimethylformamide (DMF), nitromethane, acetonitrile, and dimethylsulfoxide (DMSO), with DMSO and DMF being preferred.

The base used should be selected from the group consisting of 5 or 6 membered tertiary cyclic amines which may contain an oxygen atom, or dimethylbenzylamine. Preferable tertiary cyclic amine bases are N-methylmorpholine (NMM) and N-methylpiperidine (NMP). The base is preferably in an amount of between about 1 to 1.3 molar equivalents, and most preferably at about 1.13 molar equivalents.

The hydrochloride salt of formula (I) may be prepared as described by European Patent Application 0266896 having a publication date of May 11, 1988.

In forming the free amine (IIB) a sufficient amount of base is added to the hydrochloride salt to neutralize the compound and form the free amine. In the preferred method for producing the free amine, the hydrochloride salt (I) is neutralized by adding it to a stirred mixture of DMF of such a volume that the final solution will be

about 0.5M, and between about 1 to 1.3 equivalents of NMM, at ambient temperature. The mixing initially occurs at room temperature (20°C) for a time between about 10-20 minutes, and then it is cooled to a temperature of between about -43 to -49°C.

The mixed anhydride of formula (IIA) may be prepared by adding about 1.2 equivalents of the Dane salt of phenylglycine, below

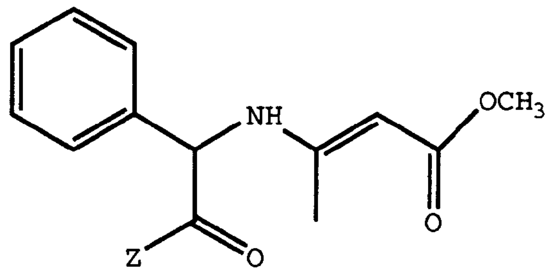

(which may be prepared according to the procedure of Dane et al., Angew. Chem., Vol. 74, 873 (1962)) wherein Z is a counterion such as sodium, lithium, potassium and ammonium, with a volume of DMF sufficient to result in a concentration of about 0.25M, and stirring the mixture at ambient temperature for 20-60 minutes. The mixture is then cooled to -43 to -49°C and NMM (0.025 equivalents) and methanesulfonic acid (0.05 equivalent) are added. Isobutyl chloroformate (1.17 equivalent) is then added and the mixture stirred for 20-30 minutes, thus resulting in the mixed anhydride (IIA).

The cooled free amine mixture (IIB) is then added to the mixed anhydride over a period of 10-20 minutes, while keeping the internal reaction temperature below -40°C. Thereafter, the mixture is warmed slowly to -28 to -32°C. An additional amount of base may be added, and warming continued to about -5°C over a one to two hour period.

The doubly blocked carbacephalosporin as represented by formula (1) can then be deprotected at both the 7-position and 4-position i.e., deprotecting the protected amino and protected carboxy group. Methods of deprotection are not limited and are illustrated by European Patent Application 266896. For example, a mixture of concentrated HCl (10.5 equivalents) in water (2:1, v:v) at a temperature at 0°-5°C, is added to the acylation solution (III) over a 30-45 minute period, during which the reaction temperature is kept below 0°C. zinc dust (3.5 equivalents) is then added over 50-70 minutes, keeping the temperature at below 0°C. Approximately 1.2 equivalents of HCl is added and the reaction mixture warmed to ambient temperature over a 45-60 minute period. The mixture is stirred for 5-6 hours at ambient temperature and semi-carbazide hydrochloride (1.15 equivalents) is added, followed by 60-75 minutes of stirring. The pH is adjusted to 2.9-3.1 with 28% aqueous ammonia and the mixture is filtered through a hyflo pad, previously washed with DMF. The filtrate is warmed to 26-30°C and is adjusted to a pH of 2.9-4.2 using 28% aqueous ammonia, a seed crystal added, and the pH is continuously adjusted to 5.9-6.4 over several hours at ambient temperature. The temperature of the mixture is lowered to 0-10°C and stirred for 50-60 minutes before being filtered, and the solid is washed with DMF.

The process disclosed results in high yields of compounds of formula III, typified by yields at over 90%.

It will be understood that the listing for substituents is exemplary and not exhaustive, and equivalents are expected to be encompassed by the spirit of the invention.

EXPERIMENTAL SECTION

Preparation 1

(R)-alpha-[(3-methoxy-1-methyl-3-oxo-1-propenyl)amino]benzene acetic acid anhydride with 2-methylpropyl hydrogen carbonate.

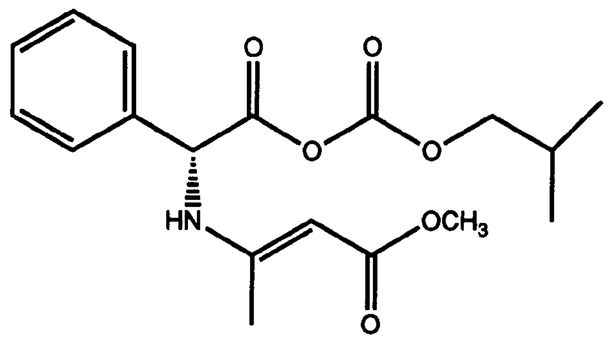

In a 100 ml 3-neck round bottomed flask, 1.50 g of the sodium Dane salt of phenylglycine and 24.6 ml of DMF were combined at room temperature. The mixture was stirred for 15 minutes under nitrogen, and then cooled to between about -44 to -50°C using a $CO_2$/acetone bath. N-methylmorpholine (0.012 ml, 0.109 mmol) was added to the mixture via syringe, followed by methanesulfonic acid (0.016 ml, 0.25 mmol), also added via syringe. Isobutyl chloroformate (0.72 g, 0.68 ml, 5.27 mmol) was then added slowly via syringe to form the mixed anhydride. A white suspension resulted and was stirred for 25 minutes at between about -44°C to -50°C under nitrogen, at which time the solid had nearly all dissolved.

Example 1

3-Chloro-7-[[[(3-methoxy-1-methyl-3-oxo-1-propenyl)aminol]phenylacetyl]amino]-8-oxo-1-azabicyclo[4.2.0]oct-2-ene-2-carboxylic acid (4-nitrophenyl)methyl ester.

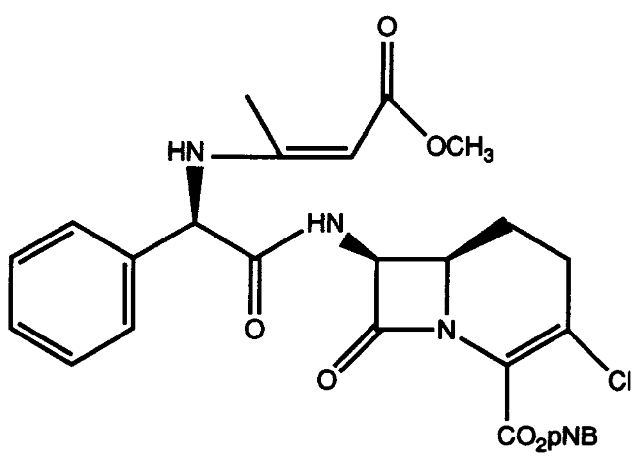

p-Nitrobenzyl 7β-amino-3-chloro-1-carba(1-dethia)-3-cephem-4-carboxylic hydrochloride salt (2.15 g, 5.27 mmol) was slurried with 10 ml DMF and N-methylmorpholine (0.58 ml, 5.27 mmol) to form the free amine. The free amine mixture was then cooled to between about -45 to -49°C.

The free amine mixture was then added dropwise with stirring under nitrogen over a period of 10 minutes to the mixed anhydride mixture from Preparation 1. Carbon dioxide was added to the cooling bath and the dropwise addition halted occasionally to maintain the temperature below -44°C. After completion of the addition, the mixture was stirred under nitrogen at between about -44 to -50°C.

After one hour the mixture was allowed to warm to -18°C over 15 minutes and another portion of N-methylmorpholine (0.064 ml) was added. Stirring continued for 15 minutes at a temperature of between about -18° to -14°C before the mixture was warmed to 0°C over a period of 10 minutes. The mixture was stirred at

0°C for 20 minutes.

Preparation 2

(R)-alpha-[(3-methoxy-1-methyl-3-oxo-1-propenyl)amino]benzene acetic acid anhydride with 2-methylpropyl hydrogen carbonate.

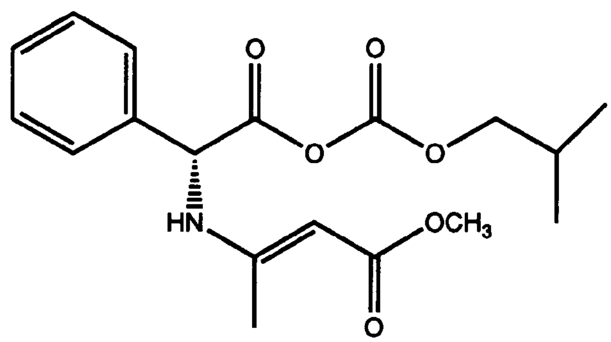

The same conditions were used as in Preparation 1 using the following amounts:

| | |
|---|---|
| Dane salt of phenyl glycine: | 1.79 g, 6.59 mmol |
| DMF: | 26.4 ml (0.25 M) |
| N-methylmorpholine: | 0.014 ml, 0.127 mmol |
| Methanesulfonic acid: | 0.017 ml, 0.262 mmoles |
| Isobutyl chloroformate: | 0.83 ml, 6.40 mmoles |

Example 2

3-Chloro-7-[[[(3-methoxy-1-methyl-3-oxo-1-propenyl)amino]phenylacetyl]amino]-8-oxo-1-azabicylco[4.2.0]oct-2-ene-2-carboxylic acid (4-nitrophenyl)methyl ester.

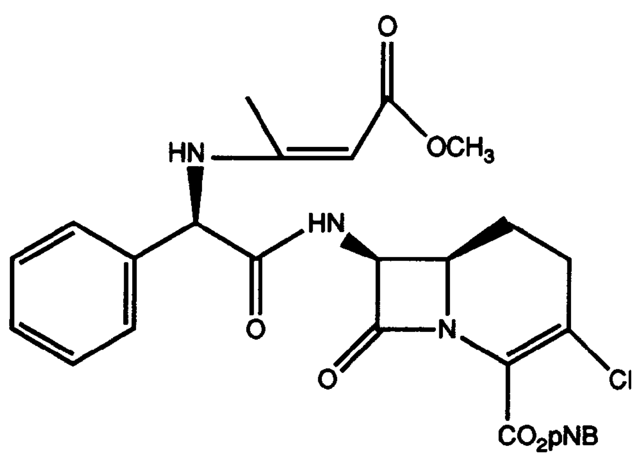

The same conditions were used as in Example 1, using the following amounts:

| | |
|---|---|
| Hydrochloride salt: | 2.40 g, 5.494 mmoles |
| DMF: | 7.5 ml, (0.72 M) |
| N-methylmorpholine | |
| (first portion): | 0.66 ml, 1.1 eq. |
| (second portion): | 0.06 ml, 0.1 eq. |

Yield of titled product - 94.5%.

Example 3

3-Chloro-7-[[[(3-methoxy-1-methyl-3-oxo-1-propenyl)amino]phenylacetyl]amino]-8-oxo-1-azabicyclo[4.2.0]oct-2-ene-2-carboxylic acid (4-nitrophenyl)methyl ester.

The sodium Dane salt of phenylglycine (20.96 g, 0.0773 mol, 1.2 eq) was placed in a one liter flask and 309 ml of DMF was added thereto. The mixture was stirred at room temperature for approximately 30 minutes. During this time, p-nitrobenzyl-7β-amino-3-chloro-1-carba(1-dethia)-3-cephem-4-carboxylic hydrochloride salt (27.68 g (90.3% pure) 0.0644 mol) was slowly added to a stirring solution of 128 ml DMF and NMM (7.40 g, 8.0 ml, 1.135 eq). After the addition of the hydrochloride salt was complete, the mixture was stirred at room temperature for 20 minutes. Both mixtures were then cooled to -45 to -49°C.

NMM (0.18 ml 0.00164 mol, 0.02 eq) and methane sulfonic acid (0.21 ml, 0.00322 mol, 0.05 eq) were added, via syringe, to the Dane salt mixture. Isobutyl chloroformate (10.38 g, 0.0760 mol, 1.18 eq) was then added to the Dane salt mixture over a period of about one minute. The internal temperature rose from -47 to -43°C after the addition was complete. The Dane salt mixture was then stirred for 30 minutes at -43 to -47°C. The hydrochloride salt mixture was then added into the Dane salt mixture over a period of about 15 minutes. The reaction mixture was stirred as the temperature rose slowly to -29°C. NMM (0.71 ml) was added to the mixture via syringe and stirring continued for another hour to a final temperature of -13°C. The bath was removed and the mixture warmed to -5°C over 15 minutes. The yield of the titled product was 91.6%.

Example 4

3-Chloro-7-[[[(3-methoxy-1-methyl-3-oxo-1-propenyl)amino]phenylacetyl]amino]-8-oxo-1-azabicyclo[4.2.0]oct-2-ene-2-carboxylic acid (4-nitrophenyl)methyl ester.

In a 250 ml 3 neck stirred flask, add 142.5 ml of DMF and 7.82 g of Nmm. Add to this mixture, in small portions and over 20 minutes with vigorous agitation, 27.5 Bgm of p-nitrobenzyl 7β-amino-3-chloro-1-carba(1-dethia)-3-cephem-4-carboxylic hydrochloride salt. Stir the reaction at about 20-25°C for 10-20 minutes and cool to -44 to -49°C.

In a stirred, 1 L 3 necked flask, add 375 ml DMF and 23 g of the sodium Dane salt of phenylglycine and stir at 20-30°C for 10-20 minutes. Cool the mixture to -44 to -49°C. To the Dane salt mixture, maintaining a temperature of -40 to -49°C, add 0.18 g of NMM and 0.341 g of methanesulfonic acid. Thereafter add quickly 11.4 g of isobutyl chloroformate. Stir the mixture at -44 to -49°C for 25 minutes.

After the 25 minute stirring, add the neutralized hydrochloride salt solution to the Dane salt mixture over a period of 10-15 minutes, maintaining a temperature of -40 to -45°C. Warm the reaction mixture over one hour to -28 to -32°C, and add 0.718 g of NMM. Warm the reaction mixture for 30 minutes at -5 to -10°C.

## Claims

1. A process for the preparation of compounds having the formula:

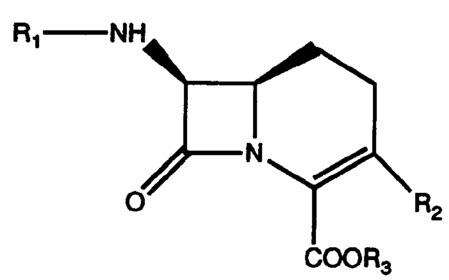

(1)

wherein $R_1$ is a group of the formula

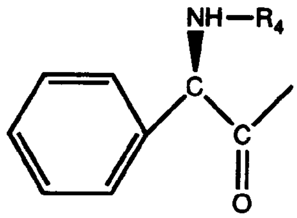

wherein $R_4$ is hydrogen or an amino-protecting group; $R_2$ is hydrogen, trihalo($C_1$-$C_4$ alkyl), $C_1$-$C_4$ alkyl, $C_1$-$C_4$ substituted alkyl, $C_1$-$C_4$ alkoxy, $C_1$-$C_4$ substituted alkoxy, $C_1$-$C_6$ alkylthio, $C_1$-$C_6$ substituted alkylthio, methoxy methyl, carbamoyloxy methyl, acetoxymethyl, $C_2$-$C_6$ alkenyl, or $C_2$-$C_6$ substituted alkenyl; and

$R_3$ is a carboxy-protecting group;

comprising the steps of mixing a compound of the formula:

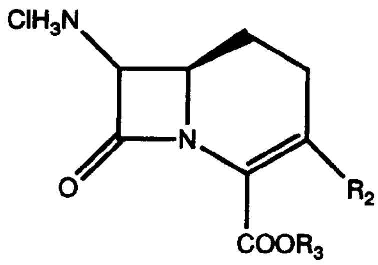

(2)

with a polar organic aprotic solvent having a dielectric constant of at least about 20 and a base selected from the group consisting of 5- or 6-membered tertiary cyclic amines containing 0 to 1 oxygen atoms, and dimethylbenzylamine, under substantially anhydrous conditions at a temperature and for a time sufficient to form the free amine base of a compound of formula (2), and thereafter, without isolating the free amine base, reacting the free amine base with an acylating agent of the formula

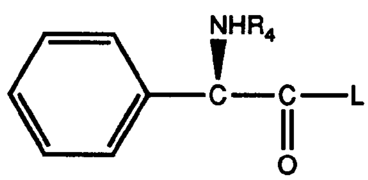

wherein L is a leaving group, under substantially anhydrous conditions and at a temperature and for a time sufficient to form a compound of formula (1).

2. The process as recited in claim 1 wherein $R_2$ is chloro.

3. The process as recited in claim 1 wherein the base is N-methylmorpholine.

4. The process as recited in claim 1 wherein L is of the formula

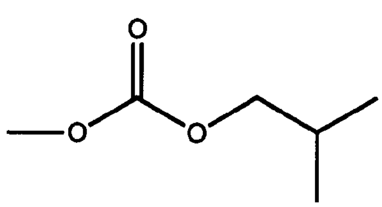

.

5. The process as recited in claim 1 wherein the step of mixing takes place at a temperature of between about -43 to -49°C.

6. The process as recited in claim 1 further comprising the step of deblocking the compound of formula (1) at either or both the 4 and 7 positions.

7. A process for the preparation of compounds having the formula

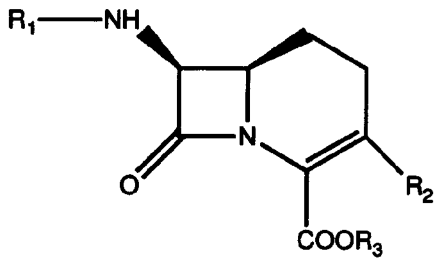

(1)

wherein $R_1$ is a group of the formula

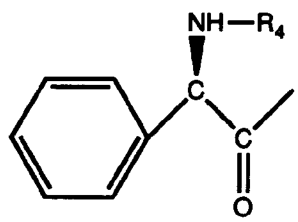

wherein $R_4$ is hydrogen or an amino-protecting group; $R_2$ is hydrogen, trihalo ($C_1$-$C_4$ alkyl) , $C_1$-$C_4$ alkyl, $C_1$-$C_4$ substituted alkyl, $C_1$-$C_4$ alkoxy, $C_1$-$C_4$ substituted alkoxy, $C_1$-$C_6$ alkylthio, $C_1$-$C_6$ substituted alkylthio, methoxy methyl, carbamoyloxy methyl, acetoxymethyl, $C_2$-$C_6$ alkenyl, or $C_2$-$C_6$ substituted alkenyl; and $R_3$ is a carboxy-protecting group; comprising the steps of mixing a compound of the formula:

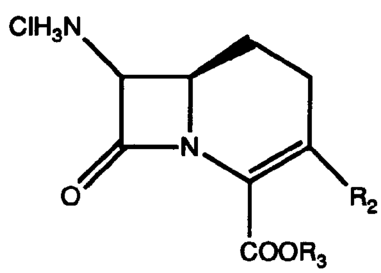

(2)

with a polar organic aprotic solvent selected from a group consisting of dimethylformamide, dimethylsulfoxide, nitromethane and acetonitrile, or a combination thereof, and a base selected from N-methylmorpholine, N-methylpiperidine and dimethylbenzylamine under substantially anhydrous conditions at a temperature and for a time sufficient to form the free amine base of a compound of formula (2), and thereafter without isolating the free amine base, reacting the free amine base under substantially anhydrous conditions with a mixed anhydride of the formula

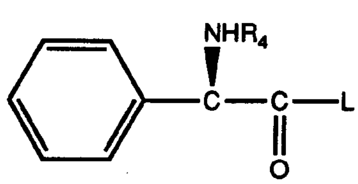

wherein L is a leaving group of the formula

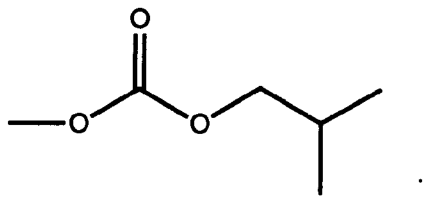

at a temperature and for a time sufficient to form a compound of formula (1).

8. The process as recited in claim 7 wherein $R_2$ is chloro.

9. The process as recited in claim 8 wherein the base is N-methylmorpholine and is in the amount of between about 1 and 1.3 molar equivalents.

10. The process as recited in claim 9 wherein the N-methylmorpholine is present in the amount of about 1.13 equivalents.

11. The process as recited in claim 9 wherein the step of mixing takes place at a temperature of between about -43 to -49°C.

12. The process as recited in claim 7 further comprising the step of deblocking the compound of formula (1) at either or both the 4 and 7 positions.